# EUROPÄISCHE PATENTSCHRIFT

(11) **EP 0 845 972 B1**
(45) Veröffentlichungstag und Bekanntmachung des Hinweises auf die Patenterteilung: **05.12.2001**
(21) Anmeldenummer: 96929256.4
(22) Anmeldetag: 14.08.1996
(51) Int. Cl.: A61K 7/00, A61K 7/48, A61K 7/06

(54) **VERWENDUNG VON MUTIERTER SUBTILISIN-PROTEASE IN KOSMETISCHEN PRODUKTEN**
USE OF MUTATED SUBTILISIN PROTEASE IN COSMETIC PRODUCTS
UTILISATION DE SUBTILYSINE PROTEASES MUTEES DANS LES PRODUITS COSMETIQUES

(30) Priorität: 23.08.1995 DE 19530816
(43) Veröffentlichungstag der Anmeldung: 10.06.1998
(73) Patentinhaber: Henkel Kommanditgesellschaft auf Aktien, 40589 Düsseldorf-Holthausen (DE)
(72) Erfinder: WEISS, Albrecht, D-40764 Langenfeld (DE); MAURER, Karl-Heinz, D-40699 Erkrath (DE)
(86) Internationale Anmeldenummer: EP9603589
(87) Internationale Veröffentlichungsnummer: WO9707770

(56) Entgegenhaltungen:
- EP-A- 0 571 049
- WO-A-92/21760
- WO-A-94/02618
- WO-A-95/10591

## Beschreibung

Die vorliegende Erfindung betrifft die Verwendung von mutierten proteolytischen Enzymen mit geringem Irritationspotential gegenüber der Haut, nämlich mutierten Subtilisin-Proteasen, in kosmetischen Produkten, insbesondere Körperreinigungs- und -pflegemitteln sowie Mundpflegemitteln.

Enzyme wie Proteasen, Lipasen, Amylasen und Cellulasen werden seit langem in Wasch- und Reinigungsmitteln im wesentlichen zur Unterstützung der Wasch- und Reinigungsleistung verwendet. Unter diesen Enzymen nehmen Proteasen eine herausragend wichtige Stellung ein.

Proteasen sind Enzyme, welche die Hydrolyse von Peptidbindungen in Protein- und Peptidsubstraten und von Esterbindungen in einigen endständigen Estern katalysieren. Subtilisine sind eine Familie von bakteriellen, extracellulären Proteasen mit Molekulargewichten von etwa 20 000 bis 45 000 Dalton, die aus Bodenbakterien, zum Beispiel Bacillus amyloliquefaciens, erhalten werden können. Subtilisine gehören zur Gruppe der Serin-Proteasen, die den nucleophilen Angriff auf die Peptid-(Ester-) Bindung über einen Serin-Rest an der aktiven Stelle initiieren. Sie sind physikalisch und chemisch gut charakterisierte Enzyme. Die dreidimensionale Struktur von einigen Subtilisinen wurde im einzelnen durch Röntgen-Beugungsaufnahmen aufgeklärt (C. Betzel, G.P. Pal und W. Saenger, (1988) Eur. J. Biochem. **178**, 155-171; R Bott, M. Ultsch, A. Kossiakoff, T. Graycar, B. Katz und S. Power, (1988) J. Biol. Chem. **263**, 7895-7906; D.W. Goddette, C. Paech, S.S. Yang, J.R Mielenz, C. Bystroff, M. Wilke und R.J. Fletterick, (1992) J. Mol. Biol. **228,** 580-595; D.W. Heinz, J.P. Priestle, J. Rahuel, K.S. Wilson und M.G. Grütter (1991) J. Mol. Biol. **217,** 353-371; J. Kraut (1977) Ann. Rev. Biochem. **46,** 331-358; D.J. Neidhart und G.A. Petsko (1988) Protein Eng. **2,** 271-276; A.V. Teplyakov, I.P. Kuranova, E.H. Harutyunyan, B.K. Vainshtein, C. Frömmel, W.-E. Höhne und K.S. Wilson (1990) J. Mol. Biol. **214**, 261-279).

Subtilisine werden in großem Umfang in handelsüblichen Produkten, wie zum Beispiel in Wasch- und Geschirrspülmitteln sowie Kontaktlinsen-Reinigungsmitteln, und in der synthetischen organischen Chemie, dort allerdings in erster Linie für Forschungszwecke, eingesetzt. Ein Mitglied der Subtilisinfamilie, nämlich eine hoch alkalische Protease, die in tensidhaltigen Mitteln verwendet werden kann, wurde in der internationalen Patentanmeldung WO 91/02792 beschrieben. Diese alkalische Protease aus Bacillus lentus (Bacillus lentus alkaline protease; BLAP) kann in kommerziell verwertbaren Mengen aus dem Stamm-Bacillus licheniformis ATCC 53926 erhalten werden, welcher ein Expressionsplasmid trägt, welches das BLAP-Gen unter der Kontrolle des Promotors der alkalischen Protease von Bacillus licheniformis ATCC 53926 exprimiert. Die Kristallstruktur von BLAP ist bestimmt worden (D.W. Goddette et al. (1992) J. Mol. Biol. 228, 580-595; WO 92/21760) und die Koordinaten wurden bei der Brookhaven Protein Datenbank hinterlegt. Wenn man eine optimale Sequenzhomologie von BLAP (269 Aminosäuren) mit der von Subtilisin BPN' (275 Aminosäuren) abgleicht, erhält man folgendes Muster: Die BLAP Positionen 1 bis 35, 36 bis 54, 55 bis 160 und 161 bis 269 entsprechen den Positionen 1 bis 35, 37 bis 55, 57 bis 162 bzw. 167 bis 275 in Subtilisin BPN'. Wenn nicht anders angegeben, entspricht die hier verwendete Numerierung der Aminosäuren der von BLAP.

Um die Protease-Varianten zu beschreiben, die in der vorliegenden Erfindung eingesetzt werden, wird die folgende Nomenclatur verwendet: [ursprüngliche Aminosäure; Position vom N-Terminus des reifen Enzyms; substituierte Aminosäure]. Zum Beispiel wird der Austausch von Valin durch Isoleucin in der Position 4 im BLAP als V4I bezeichnet. Die Liste der üblichen Abkürzungen für die gebräuchlichen Aminosäuren ist in Tabelle 1 dargestellt.

**Tabelle 1:**

| Abkürzung der Aminosäuren |
|---|
| A = Ala = Alanin |
| C = Cys = Cystein |
| D = Asp = Asparaginsäure |
| E = Glu = Glutaminsäure |
| F = Phe = Phenylalanin |
| G = Gly = Glycin |
| H = His = Histidin |
| I = Ile = Isoleucin |
| K = Lys = Lysin |
| L = Leu = Leucin |
| M = Met = Methionin |
| N = Asn = Asparagin |
| P = Pro = Prolin |
| Q = Gln = Glutamin |
| R = Arg = Arginin |
| S = Ser = Serin |
| T = Thr = Threonin |
| V = Val = Valin |
| W = Trp = Tryptophan |
| Y = Tyr = Tyrosin |

Beim Auftreten mehrerer Mutationen innerhalb desselben Protein-Moleküls wird dieses über die Summe der einzelnen Mutationen charakterisiert, wie zum Beispiel S3T + V4I + A188P + V193M + V199I.

Der Schutz gegenüber thermischer und chemischer Inaktivierung und die Verbesserung der Wasch- und Reinigungswirkung sowie der Hautverträglichkeit sind primäre Aufgaben, wenn man neue Proteasen für technische und gewerbliche Anwendungen entwickeln will. Eine Vielzahl von Enzymen, darunter auch Proteasen vom Subtilisin-Typ, wurden durch Zufallsmutagenese oder ortsspezifische Mutagenese entwickelt. Sie liefern einige Hinweise, wie man eine verbesserte thermische und chemische Stabilität auf rationalem Wege erreichen kann (D.A. Estell, T.P. Graycar und J.A. Wells (1985) J. Biol. Chem. **260,** 6518-6521; M. Matsumura, W.J. Becktel, M. Levitt und B.W. Matthews (1989) Proc. Natl. Sci. US **86,** 6562-6566; M.W. Pantoliano, M. Whitlow, J.F. Wood, M.L. Rollence, B.C. Finzel, G.L. Gilliland, T.L. Poulos und P.N. Bryan (1988) Biochemistry **27,** 8311-8317; A.J. Russell und A.R. Fersht (1987) Nature **328,** 496-500; R.J. Siezen, W.M. De Vos, J.A.M. Leunissen, und B.W. Dijkstra (1991) Protein Eng. **4**, 719-737; J.H. van Ee (1991) Chimicaoggi (**7/8**), 31-35; J.A. Wells und D.A. Estell (1988) Trends Biochem. Sci. **13**, 291-287). Demgegenüber ist die Veränderung der enzymatischen Aktivität, insbesondere der Verbesserung oder Optimierung der Geschwindigkeit für einige Substrate, ein wesentlich komplexeres Problem. EP 0 260 105 offenbart die Herstellung von Subtilisin-BPN'-Mutanten mit veränderten Verhältnissen von Umesterungsgeschwindigkeit zu Hydrolysegeschwindigkeit und nucleophilen Spezifitäten durch Verändern spezifischer Aminosäurereste innerhalb von 15 Å der katalytischen Triade. A.J. Russell und A.R. Fersht (1987) J. Mol. Biol. 193, 803-813, beschreiben die Isolierung einer Subtilisin-BPN'-Mutante (D099S), die eine Veränderung der Oberflächenladung im Abstand von 14 bis 15 Å vom aktiven Zentrum aufweist. Diese Substitution beeinflußt die pH-Abhängigkeit der katalytischen Reaktion des Subtilisins. Keine dieser Veröffentlichungen lehrt, ob die Veränderungen der Aminosäuren auch eine Veränderung der Hautverträglichkeit der Enzyme bewirken. EP 0 130 756, EP 0 247 647 und US 4 760 025 offenbaren ein Sättigungsmutationsverfahren, worin mindestens eine Mutation in das Subtilisin BPN' an den Aminosäureresten (BPN'-Numerierung) Asp32, Asn155, Tyr104, Met222, Gly166, His64, Ser221, Gly169, Glu156, Ser33, Phe189, Tyr217 und/oder Ala152 eingeführt wird. Unter Verwendung dieser Vorgehensweise werden mutierte Proteasen, die eine verbesserte oxidative Stabilität, eine veränderte Substratspezifität und/oder eine veränderte pH-Aktivität zeigen, erhalten. Diese Veröffentlichungen lehren auch, daß Mutationen im Bereich des aktiven Zentrums der Protease die Aktivität am meisten beinflussen. Jedoch offenbart keine dieser Schriften ein Verfahren, das es ermöglicht, vorherzusagen, ob und welche Veränderungen in der Aminosäuresequenz die Hautverträglichkeit von Proteasen verbessern.

Die meisten der Informationen über die katalytische Aktivität von Subtilisinen wurden bei Untersuchungen der Hydrolyse kleiner, gut definierter Peptidsubstrate gesammelt. Bis jetzt ist nur wenig über Interaktionen mit großen Proteinsubstraten bekannt. Dies gilt insbesondere für die Informationen zur Waschleistung von Proteasen, wenn deren Substrat an eine textile Oberfläche gebunden ist und die Katalyse in Gegenwart von mit dem Enzym wechselwirkenden Substanzen wie Bleichmitteln, Tensiden und Buildern stattfinden muß. Auch ist nichts über die Wechselwirkung der Proteasen mit den in Haut- und Haarpflegemitteln sowie Mundpflegemitteln üblicherweise enthaltenen Substanzen bekannt.

EP 0 328 229 offenbart die Isolierung und Charakterisierung von PB92 Subtilisin-Mutanten mit verbesserten Eigenschaften bei der Anwendung in Waschmitteln, basierend auf den Ergebnissen von Waschtests. Dieses Dokument lehrt, daß biochemische Eigenschaften keine verläßlichen Parameter sind, um die Enzymleistung bei der Wäsche vorauszusagen. Dort vorgestellte Verfahren zur Auswahl von Mutationen beinhalten die Substitution von Aminosäuren durch andere Aminosäuren in der gleichen Kategorie (polar, unpolar, aromatisch, geladen, aliphatisch und neutral), die Substitution von polaren Aminosäuren wie Asparagin und Glutamin durch geladene Aminosäuren, und das Erhöhen des anionischen Charakters der Protease an den Mutationsstellen, die nicht zu den aktiven Zentren gehören. Es wird kein Verfahren beschrieben, mit welchem identifiziert werden kann, welche spezifischen Aminosäuren verändert werden sollten.

Es sind mehrere Patentanmeldungen bekannt, in denen Modifikationen von Subtilisin-Enzymen beschrieben werden, um deren Wirkungsweise in Waschmitteln zu verbessern, zum Beispiel die internationalen Patentanmeldungen WO 91/00345 und WO 92/11357.

Die europäische Patentanmeldung EP 0 571 049 offenbart bestimmte mutierte proteolytische Enzyme. Diese Enzyme sollen zu mindestens 70 % homolog sein mit der Aminosäure-Sequenz der PB92-Serin-Protease und sich von der PB92-Serin-Protease durch mindestens eine Aminosäure an den Positionen 99, 102, 116, 126, 127, 128, 130, 160, 203, 211 und/oder 212 unterscheiden. Die mutierte Protease wird durch Züchten eines Wirtstammes, welcher mit einem Expressionsvektor transformiert wurde, der eine DNA-Sequenz enthält und für die gewünschte mutierte Protease codiert, hergestellt.

In der nicht vorveröffentlichten internationalen Patentanmeldung WO 95/23221 werden mutierte Proteasen beschrieben, die als Zusatz zu Wasch- und Reinigungsmitteln deren Wirksamkeit verbessern.

Die WO-A-95 10591 offenbart ein Reinigungsmittel mit einem Gehalt an einer Protease, wobei proteolytische Aktivität, Substratspezifität und Stabilität erhöht sind.

Der vorliegenden Erfindung lag die Aufgabe zugrunde, mutierte Proteasen zur Verfügung zu stellen, die gegenüber der ursprünglichen Protease verbesserte Hautverträglichkeiten aufweisen und in in kosmetischen Produkten, insbesondere Körperreinigungs- und pflegemitteln sowie Mundpflegemitteln, eingesetzt werden können.

Überraschenderweise wurde gefunden, daß insbesondere die in WO 95/23221 genannten mutierten Proteasen diese Forderung erfüllen Gegenstand der vorliegenden Erfindung ist die Verwendung von mutierter Subtilisin-Protease in kosmetischen Produkten, welche dadurch gekennzeichnet ist, daß die mutierte Subtilisin-Protease mindestens eine Mutation in ihrer Aminsosäure-Sequenz enthält, die zu einer reduzierten positiven Ladung oder einer erhöhten negativen Ladung in der Nähe der an das Substrat gebundenen Region des Moleküls ("Substratbindungsstelle") führt.

Zu den kosmetischen Produkten, in denen die mutierte Protease erfindungsgemäß eingesetzt werden kann, sind insbesondere Körperreinigungs- und -pflegemittel sowie Mundpflegemittel zu nennen, wie zum Beispiel Seifen in fester und flüssiger Form, Peeling-Cremes, Hautcremes, Softcremes, Nährcremes, Sonnenschutzcremes, Nachtcremes, Hautöle, Pflegelotionen und Körper-Aerosole, Deodorants, Rasiercremes und Rasierschäume, Haarshampoos, Haarspülungen und Schaumbäder, Mundspülungen und Zahnpasten.

Die erfindungsgemäß zu verwendenden mutierten Proteasen zeigen überraschenderweise ein geringes Irritationspotential gegenüber der Haut und den Schleimhäuten und eignen sich demgemäß hervorragend zum Einsatz in den voranstehend genannten Produkten.

Ein weiterer Gegenstand der Erfindung betrifft die Verwendung der voranstehend genannten Proteasen zur Herstellung von Mitteln zur Haut-, Haar- und Körperpflege. Zur Herstellung dieser Mittel werden die einzelnen Komponenten in an sich bekannter Weise vermischt. Die Mittel können, insbesondere wenn sie lipophile Stoffe enthalten, sowohl als "Wasser-in-Öl" als auch "Öl-in-Wasser"- Emulsionen vorliegen und weitere übliche Hilfs- und Zusatzstoffe enthalten.

Die Mittel können neben den erfindungsgemäß eingesetzten Proteasen als wesentliche Bestandteile insbesondere Tenside, wie anionische, nichtionische, kationische, amphotere und/oder zwitterionische Tenside, enthalten.

Als Hilfs- und Zusatzstoffe eignen sich beispielsweise Emulgatoren, Ölkomponenten, Fette und Wachse, Verdickungsmittel, Überfettungsmittel, biogene Wirkstoffe, Filmbildner, Duftstoffe, Farbstoffe, Perglanzmittel, Konservierungsmittel und pH-Regulatoren.

Unter den üblichen Ölkomponenten sind Substanzen wie Paraffinöl, Pflanzenöle, Fettsäureester, Siliconöle, Dialkylether, Fettalkohole und Guerbetalkohole, Squalan und 2-Octyldodecanol zu nennen, während als Fette und Wachse beispielsweise Walrat, Bienenwachs, Montanwachs, Paraffin und Cetylstearylalkohol Verwendung finden.

Als Emulgatoren können beispielsweise eingesetzt werden: Sorbitanester, Monoglyceride, Polysorbate, Polyethylenglycolmono/difettsäureester, hochethoxylierte Fettsäureester sowie hochmolekulare Siliconverbindungen, wie zum Beispiel Dimethylpolysiloxane mit einem durchschnittlichen Molekulargewicht von 10.000 bis 50.000.

Als Überfettungsmittel können Substanzen wie polyethoxylierte Lanolin-Derivate, Dicytin-Derivate und Fettsäurealkanolamide verwendet werden, wobei die letzteren gleichzeitig als Schaumstabilisatoren dienen.

Geeignete Verdickungsmittel sind beispielsweise Polysaccharide, insbesondere Xanthan-Gummi, Guar, Agar-Agar, Alginate und Tylosen, Carboxymethylcellulose und Hydroxyethylcellulose, ferner höher molekulare Polyethylenglykolmono- und -diester von Fettsäuren, Polyacrylate, Polyvinylalkohol und Polyvinylpyrolidon sowie Elektrolyte wie Kochsalz und Ammoniumchlorid.

Unter biogenen Wirkstoffen sind beispielsweise Pflanzenextrakte, Eiweißhydrolysate und Vitaminkomplexe zu verstehen.

Gebräuchliche Filmbildner sind beispielsweise Polyvinylpyrolidon, Vinylpyrolidon-Vinylacetat-Copolymerisate, Polymere der Acrylsäurereihe, quaternäre Cellulose-Derivate und ähnliche Verbindungen.

Als Konservierungsmittel eignen sich beispielsweise Formaldehyd-Lösungen, p-Hydroxybenzoesäureester oder Sorbinsäure.

Als Perglanzmittel kommen beispielsweise Glykoldistearinsäureester wie Ethylenglykoldistearat, aber auch Fettsäuremonoglykolester in Betracht.

Als Farbstoffe können die für kosmetische Zwecke geeigneten und zugelassenen Substanzen verwendet werden, wie sie beispielsweise in der Publikation "Kosmetische Färbemittel" der Farbstoffkommission der deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen auf die gesamte Mischung, eingesetzt.

Insbesondere in Cremes können, gegebenenfalls neben den bereits genannten Zusätzen, auch Antioxidantien, wie zum Beispiel Butylhydroxytoluol und Tocopherol, Feuchthaltemittel, wie zum Beispiel Glycerin, Sorbitol, 2-Pyrrolidin-5-carboxylat, Dibutylphthalat, Gelatine, Polyglycole mit einem durchschnittlichen Molekulargewicht von 200 bis 600, pH-Puffersubstanzen, wie zum Beispiel das System Milchsäure/Triethanolamin oder Milchsäure/NaOH, milde Tenside, wie zum Beispiel Alkyloligoglucoside, Fettalkoholethersulfate, Fettsäureisethionate, -tauride und -sarcosinate, Ethercarbonsäuren, Sulfosuccinate, Eiweißhydrolysate beziehungsweise -fettsäurekondensate, Sulfotriglyceride, kurzkettige Glucamide, Phospholipide, Pflanzenextrakte, zum Beispiel von Aloe vera, Sonnenschutzmittel, wie zum Beispiel ultrafeines Titandioxid oder organische Stoffe wie p-Aminobenzoesäure und deren Ester, Ethylhexyl-p-methoxyzimtsäureester, 2-Ethoxyethyl-p-methoxyzimtsäureester, Butylmethoxydibenzoylmethan und deren Mischungen sowie sogenannte Wirkstoffbeschleuniger, insbesondere etherische Öle, wie beispielsweise Eucalyptol, Menthol und ähnliche enthalten sein.

Um Proteasen in Mitteln der voranstehend beschriebenen Art einsetzen zu können, dürfen sie keine oder nur geringe Irritationen gegenüber der Haut beziehungsweise den Schleimhäuten hervorrufen.

Der ursprüngliche Proteasetyp, von welchem sich die gemäß der Erfindung zu verwendenden mutierten Proteasen ableiten, ist vorzugsweise eine oben beschriebene alkalische Bacillus lentus Protease (BLAP), die aus dem Stamm DSM 5483 erhalten wird und 269 Aminosäureeinheiten, eine Molekülmasse von 26.823 Dalton und einen berechneten isoelektrischen Punkt von 9,7, bezogen auf pK-Standardwerte, aufweist. Das BLAP-Gen kann in bekannter Weise durch Isolieren der chromosomalen DNA aus dem B. lentus-Stamm DSM 5483, Herstellen von DNA-Proben mit einer Homologie gegenüber den DNA-Sequenzen der für die B. lentus Protease codierenden Regionen, Herstellen von Genom-Bibliotheken aus der isolierten chromosomalen DNA und Auswählen der Bibliotheken für die interessierenden Gene durch Hybridisierung der Proben erhalten werden. Mutanten des genannten BLAP mit verbesserter Stabilität gegenüber thermischer Belastung und Tensiden wurden in der internationalen Patentanmeldung WO 92/21760 beschrieben.

Wie nun gefunden wurde, kann ein verringertes Irritationspotential gegenüber der Haut und den Schleimhäuten erzielt werden, wenn Aminosäure-Veränderungen innerhalb des Substratbindungsbereichs des Enzyms durchgeführt werden, die zu einer Erhöhung der negativen Ladung führen. Gemäß der vorliegenden Erfindung kann dies beispielsweise durch eine Vermehrung von negativ geladenen Aminosäureresten oder einer Reduktion von positiv geladenen Aminosäureresten im Substratbindungsbereich im Umkreis von 7 Å erfolgen, wobei die Substratbindungsstelle mit Hilfe eines gebundenen Substratmoleküls, wie zum Beispiel AAPF, festgestellt werden kann. Insbesondere Aminosäure-Veränderungen an den Positionen 99, 154 und 211 innerhalb der aus WO 94/21760 bekannten BLAP-Varianten M130 und M131 von Bacillus lentus führen zu einem verringerten Irritationspotential gegenüber Haut und Schleimhäuten. Die Mutante M130 enthält vier Aminosäure-Veränderungen im Vergleich zu nativem BLAP: S3T, A188P, V193M und V199I. Die Mutante M131 enthält fünf Aminosäure-Veränderungen im Vergleich zu nativem BLAP: S3T, V4I, A188P, V193M und V199I. Die Aminsosäure-Sequenz für die Protease M130 beziehungsweise M131 ist in der SEQ ID No.:2 beziehungsweise SEQ ID No.:1 der genannten internationalen Patentanmeldung WO 92/21760 wiedergegeben. M130 und M131 können als Basis für weitere Aminsoäuren-Veränderungen dienen, um Proteasen mit verringertem Irritationspotential zu erhalten. Bevorzugte Proteasemutanten zur erfindungsgemäßen Verwendung sind solche, die durch Ersatz mindestens einer Aminosäure der Proteasen M130 oder M131 erhalten werden, worin die Aminosäure ausgewählt wird aus der Gruppe bestehend aus Arginin an Position 99, Serin an Position 154 und Leucin an Position 211. Derartige Mutanten und ihre Herstellung sind in der internationalen Patentanmeldung WO 95/21221 offenbart und werden dort als F11 (S3T + R99S + A188P + V193M + V199I), F43 (S3T + V4I + R99G + A188P + V193M + V199I), F44 (S3T + V4I + R99A + A188P + V193M + V199I), F45 (S3T + V4I + R99S + A188P + V193M + V199I), F46 (S3T + V4I + S154E + A188P + V193M + V199I), F47 (S3T + V4I + S154D + A188P + V193M + V199I), F49 (S3T + V4I + A188P + V193M + V199I + L211D), F54 (S3T + V4I + R99G + A188P + V193 + V199I + L211D) und F55 (S3T + V4I + S154D + A188P + V193M + V199I + L211D) bezeichnet. Die proteolytische Aktivität kann in Anlehnung an die in Tenside 7 (1970), 125 beschriebene Methode durch eine diskontinuierliche Bestimmung unter Verwendung von Casein als Substrat wie folgt gemessen werden: Die Konzentrationen der Substrat-Lösung betragen 12 mg pro ml Casein (hergestellt gemäß Hammarsten; Lieferant: Fa. Merck, Darmstadt, Nr. 2242) und 30 mM Tris in synthetischem Leitungswasser (wäßrige Lösung von 0,029 % (Gew./V) CaCl₂ · 2H₂O, 0,014 % (Gew./V) MgCl₂ · 6H₂O und 0,021 % (Gew./V.) NaHCO₃) mit einer Härte von 15 Grad dH (deutsche Härte). Die Substrat-Lösung wird auf 70° C erwärmt und der pH wird mit 0,1 N NaOH bei 50° C auf pH 8,5 eingestellt. Die Protease-Lösung wird mit 2 % (Gew./V) wasserfreiem Pentanatrium-Tripolyphosphat in synthetischem Leitungswasser hergestellt, wobei der pH mit Salzsäure auf 8,5 eingestellt wird. Zu 600 µl der Casein-Substrat-Lösung werden 200 µl der Enzym-Lösung zugegeben. Das Gemisch wird 15 Minuten bei 50° C inkubiert. Die Reaktion wird durch Zugabe von 600 µl 0,44 M Trichloressigsäure (TCA), 0,22 M Natriumacetat in 3 % (V/V) Essigsäure beendet. Nach Abkühlen auf Eis innerhalb von 15 Minuten wird das TCA-unlösliche Protein durch Zentrifugieren entfernt, ein Aliquot von 900 µl wird mit 300 µl 2 N NaOH gemischt und die Extinktion dieses Gemisches, das TCA-lösliche Peptide enthält, wird bei 290 nm aufgenommen. Kontrollwerte werden hergestellt durch Hinzufügen von 600 µl der TCA-Lösung zu 600 µl Casein-Lösung gefolgt von 200 µl Enzym-Lösung. Definitionsgemäß besitzt eine Protease-Lösung, die unter den Bedingungen dieses Versuchs eine Extinktionsänderung von 0,500 OD bei 290 nm hervorruft, eine Aktivität von 10 PE pro ml.

### Beispiele

### Beispiel 1: Bestimmung des Irritationspotentials

Die Irritationspotentiale erfindungsgemäß einzusetzender Proteasen am Beispiel von F49 und zum Vergleich von BLAP wurden untersucht. Es wurde an Meerschweinchen im Rahmen von Dosisfindungsversuchen des Buehler-Tests bestimmt (E.V. Buehler, Arch. Dermatol. 1965, 91, 171-177). Hierzu wurde eine 10%ige Lösung der Protease auf die rasierte Haut topisch appliziert und mittels einer Bandage (Scotchpak® -Non-Woven-Patch, Handelsprodukt; Hersteller Minnesota Mining and Manufacturing Company) für sechs Stunden fixidert. 24, 48 und 72 Stunden nach der Entfernung der Bandagen wurden die betreffenden Hautpartien ausgewertet.

Diese Versuche wurden an jeweils vier Meerschweinchen (Stamm: "Pirbright white") durchgeführt, wobei pro Tier die Lösungen an mindestens zwei Applikationsstellen aufgebracht wurden. Die Ergebnisse sind in den folgenden Tabellen 2 und 3 wiedergegeben und stellen Mittelwerte über Erythem- und Ödembildung dar. Die Ergebnisse zeigen, daß die erfindungsgemäß einzusetzende mutierte Protease ein deutlich geringeres Irriationspotential zeigt als die unveränderte Protease BLAP.

In den Tabellen bedeuten
- die Ziffern 0, 1 und 2: die Anzahl Hautbereiche an einem Tier, an denen Irritationen der Haut beobachtet wurden,
- s: leichte Schorfbildung am Rand der behandelten Hautbereiche,
- a: Schwellung

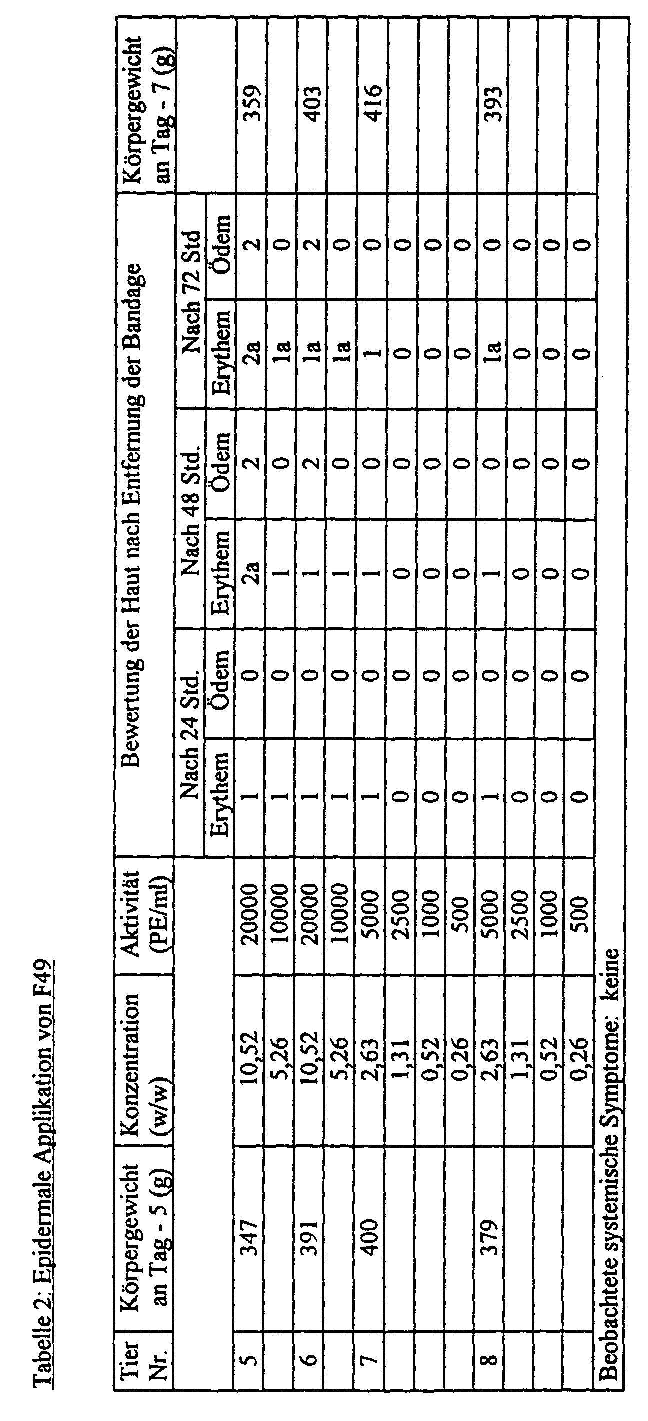

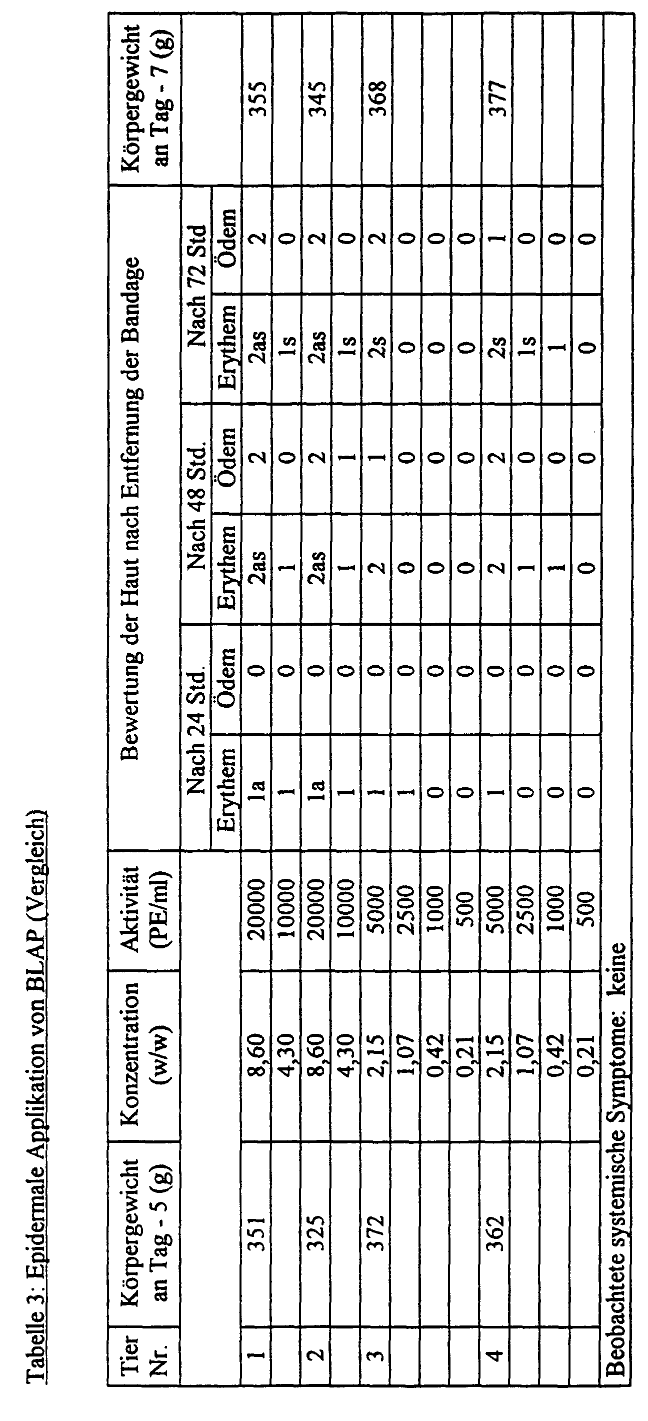

## Patentansprüche

1. Verwendung von mutierter Protease vom Subtilisin-Typ, die mindestens eine Mutation in ihrer Aminosäure-Sequenz trägt, die zu einer reduzierten positiven Ladung oder einer erhöhten negativen Ladung in der Substrat-Bindungsregion des Moleküls führt, in kosmetischen Produkten.

2. Verwendung nach Anspruch 1, **dadurch gekennzeichnet, daß** sich die mutierte Protease von alkalischer Bacillus lentus Protease ableitet.

3. Verwendung nach Anspruch 2, **dadurch gekennzeichnet, daß** die mutierte Protease eine mutierte alkalische Protease M131 (S3T + V4I + A188P + V193M + V199I) oder eine mutierte alkalische Protease M130 (S3T + A188P + V193M +V199I) ist.

4. Verwendung nach Anspruch 2 oder 3, **dadurch gekennzeichnet, daß** die Protease mindestens eine Mutation in ihrer Aminosäuresequenz ausgewählt aus den Positionen 99, 154 und 211 aufweist.

5. Verwendung nach einem der Ansprüche 2 bis 4, **dadurch gekennzeichnet, daß** der Aminosäurerest 99, Arginin, durch einen Glycin-, Alanin- oder Serinrest ersetzt ist.

6. Verwendung nach einem der Ansprüche 2 bis 5, **dadurch gekennzeichnet, daß** der Aminosäurerest 154, Serin, durch einen Glutaminsäure- oder einen Asparaginsäurerest ersetzt ist.

7. Verwendung nach einem der Ansprüche 2 bis 6, **dadurch gekennzeichnet, daß** der Aminosäurerest 211, Leucin, durch einen Glutaminsäure- oder einen Asparaginsäurerest ersetzt ist.

8. Verwendung nach einem der Ansprüche 1 bis 7, **dadurch gekennzeichnet, daß** die mutierte Protease eine oder mehrere der Mutationen R99G, R99S, R99A, L211D, L211E, S154D, S154E aufweist.

9. Verwendung nach einem der Ansprüche 1 bis 8, **dadurch gekennzeichnet, daß** die kosmetischen Produkte ausgewählt werden aus Seifen in fester und flüssiger Form, Hautcremes, Softcremes, Nährcremes, Sonnenschutzcremes, Nachtcremes, Hautölen, Pflegelotionen, Körper-Aerosolen, Deodorants, Rasiercremes, Rasierschäumen, Peeling-Cremes, Haarshampoos, Haarspülungen, Schaumbäder, Mundspülungen und Zahnpasten.

10. Verfahren zur Herstellung von kosmetischen Produkten, **dadurch gekennzeichnet, daß** man mutierte Protease vom Subtilisin-Typ, die mindestens eine Mutation an ihrer Aminosäure-Sequenz trägt, die zu einer reduzierten positiven Ladung oder einer erhöhten negativen Ladung in der Substrat-Bindungsregion des Moleküls führt, mit weiteren Inhaltsstoffen kosmetischer Produkte vermischt.

11. Verfahren nach Anspruch 10, **dadurch gekennzeichnet, daß** als weitere Inhaltsstoffe anionische, nichtionische, kationische, amphotere beziehungsweise zwitterionische Tenside, Emulgatoren, Ölkomponenten, Fette und Wachse, Verdickungsmittel, Überfettungsmittel, biogene Wirkstoffe, Filmbildner, Duftstoffe, Farbstoffe, Perglanzmittel, Konservierungsmittel und/oder pH-Regulatoren eingesetzt werden.

## Claims

1. The use of mutated protease of the subtilisin type containing at least one mutation in its amino acid sequence which leads to a reduced positive charge or to an increased negative charge in the substrate-binding region of the molecule in cosmetic products.

2. The use claimed in claim 1, **characterized in that** the mutated protease is derived from alkaline Bacillus lentus protease.

3. The use claimed in claim 2, **characterized in that** the mutated protease is a mutated alkaline protease M131 (S3T + V41 + A188P + V193M +V199I) or a mutated alkaline protease M130 (S3T + A188P + V193M +V199I).

4. The use claimed in claim 2 or 3, **characterized in that** the protease has at least one mutation in its amino acid sequence selected from positions 99, 154 and 211.

5. The use claimed in any of claims 2 to 4, **characterized in that** the amino acid residue 99, arginine, is replaced by a glycine, alanine or serine residue.

6. The use claimed in any of claims 2 to 5, **characterized in that** the amino acid residue 154, serine, is replaced by a glutamic acid or an aspartic acid residue.

7. The use claimed in any of claims 2 to 6, **characterized in that** the amino acid residue 211, leucine, is replaced by a glutamic acid or an aspartic acid residue.

8. The use claimed in any of claims 1 to 7, **characterized in that** the mutated protease has one or more of the mutations R99G, R99S, R99A, L211D, L211E, S154D, S154E.

9. The use claimed in any of claims 1 to 8, **characterized in that** the cosmetic products are selected from soaps in solid and liquid form, skin cremes, soft cremes, nourishing cremes, sun protection cremes, night cremes, skin oils, skin care lotions, body aerosols, deodorants, shaving cremes, shaving foams, peeling cremes, hair shampoos, hair rinses, foam baths, mouthwashes and toothpastes.

10. A process for the production of cosmetic products, **characterized in that** mutated protease of the subtilisin type containing at least one mutation in its amino acid sequence which leads to a reduced positive charge or to an increased negative charge in the substrate-binding region of the molecule is mixed with other ingredients of cosmetic products.

11. A process as claimed in claim 10, **characterized in that** anionic, nonionic, cationic, amphoteric or zwitterionic surfactants, emulsifiers, oil components, fats and waxes, thickeners, superfatting agents, biogenic agents, film formers, fragrances, dyes, pearlescers, preservatives and/or pH regulators are used as further ingredients.

## Revendications

1. Utilisation d'une protéase mutée du type subtilisine, portant au moins une mutation dans sa séquence d'aminoacides, qui conduit à une réduction de la charge positive ou à une augmentation de la charge négative dans la région de liaison du substrat, de la molécule dans des produits cosmétiques.

2. Utilisation selon la revendication 1,
**caractérisée en ce que**
la protéase mutée dérive d'une protéase alcaline de Bacillus lentus.

3. Utilisation selon la revendication 2,
**caractérisée en ce que**
la protéase mutée est une protéase alcaline mutée M131 (S3T + V41 + A188P + V193M + V199I) ou une protéase alcaline mutée M130 (S3T + A188P + V193M + V199I).

4. Utilisation selon l'une des revendications 2 ou 3,
**caractérisée en ce que**
la protéase possède au moins une mutation dans sa séquence d'aminoacides choisie parmi les positions 99, 154 et 211.

5. Utilisation selon l'une des revendications 2 à 4,
**caractérisée en ce que**
le reste d'aminoacide en 99, l'arginine, est remplacé par un reste glycine, alanine ou sérine.

6. Utilisation selon l'une des revendications 2 à 5,
**caractérisée en ce que**
le reste d'aminoacide en 154, la sérine, est remplacé par un reste acide glutamique ou un reste acide aspartique.

7. Utilisation selon l'une des revendications 2 à 6
**caractérisée en ce que**
le reste d'amino acide en 211, la leucine, est remplacé par un reste acide glutamique ou par un reste acide aspartique

8. Utilisation selon l'une des revendications 1 à 7,
**caractérisée en ce que**
la protéase mutée possède une ou plusieurs des mutations R99G, R99S, R99A, L211D, L211E, S154D, S154E.

9. Utilisation selon l'une des revendications 1 à 8,
**caractérisée en ce que**
les produits cosmétiques sont choisis parmi les savons sous forme solide et liquide, les crèmes pour la peau, les crèmes souples, les crèmes nourrissantes, les crèmes protectrices contre le soleil, les crèmes de nuit, les huiles pour la peau, les lotions de soins, les aérosols pour le corps, les déodorants, les crèmes à raser, les mousses à raser, les crèmes pour le peeling, les shampooings capillaires, les produits de rinçage pour les cheveux, les bains moussants, les bains de rinçage pour la bouche, et les pâtes dentifrices.

10. Procédé de préparation de produits cosmétiques,
**caractérisé en ce qu'**
on mélange une protéase mutée du type subtilisine qui porte au moins une mutation sur la séquence d'aminoacides qui entraîne une réduction de la charge positive ou une élévation de la charge négative dans la région de liaison du substrat de la molécule, avec d'autres ingrédients de produits cosmétiques.

11. Procédé selon la revendication 10,
**caractérisé en ce que**
comme autres ingrédients on met en oeuvre des agents tensioactifs anioniques, cationiques, amphotères ou zwitterioniques, des agents émulsionnants, des composants huileux, des graisses et des cires, des agents épaississants, des produits de surgraissage, des principes actifs biogènes, des agents filmogènes, des parfums, des colorants, des produits qui donnent un éclat nacré, des agents conservateurs et/ou des régulateurs de pH.
